Europäisches Patentamt

(19) European Patent Office

Office européen des brevets

(11) Veröffentlichungsnummer: **0 009 288**
**B1**

(12) EUROPÄISCHE PATENTSCHRIFT

(45) Veröffentlichungstag der Patentschrift:
20.01.82

(21) Anmeldenummer: 79200511.8

(22) Anmeldetag: 14.09.79

(51) Int. Cl.³: **G 01 N 24/06, H 01 F 7/20,**
**A 61 B 5/05**

(54) Magnetspulenanordnung zur Erzeugung von linearen magnetischen Gradientenfeldern.

(30) Priorität: 15.09.78 DE 2840178

(43) Veröffentlichungstag der Anmeldung:
02.04.80 Patentblatt 80/7

(45) Bekanntmachung des Hinweises auf die Patenterteilung:
20.01.82 Patentblatt 82/3

(84) Benannte Vertragsstaaten:
BE DE FR GB IT NL

(56) Entgegenhaltungen:
DE-A-1 946 059
FR-A-2 311 299
US-A-3 582 779
US-A-4 095 588

ELECTRO/78 Conf.-Record, Mai 1978,
CHING-MING LAI, WAYLON V. HOUSE Jr.
und PAUL C. LAUTERBUR: «Nuclear
magnetic resonance zeugmatography
of medical imaging», Seiten 1–15

JOURNAL OF PHYSICS E; scientific
insturments; Band 9, Nr. 21,
Januar 1976.
ZUPANCIC und PIRS: «Coils producing

(73) Patentinhaber: **Philips Patentverwaltung GmbH,**
**Steindamm 94, D-2000 Hamburg 1 (DE)**

(84) Benannte Vertragsstaaten: **DE**

(73) Patentinhaber: **N.V. Philips' Gloeilampenfabrieken, Pieter**
**Zeemanstraat 6, NL-5621 CT Eindhoven (NL)**

(84) Benannte Vertragsstaaten: **BE FR GB IT NL**

(72) Erfinder: **Heinzerling, Jürgen, Dr.rer.nat.,**
**Pezolddamm 42, D-2000 Hamburg 71 (DE)**

(74) Vertreter: **Hartmann, Heinrich et al, Philips**
**Patentverwaltung GmbH Steindamm 94,**
**D-2000 Hamburg 1 (DE)**

(56) Entgegenhaltungen:

a magnetic field gradient for diffusion
measurements with NMR»,
Seiten 79, 80

REVIEW OF SCIENTIFIC INSTRUMENTS,
Band 45, Nr. 10, Oktober 1974,
D.S. WEBSTER und K.H. MARSDEN:
«Improved apparatus for the NMR
measurement of self-diffusion
coefficients using pulsed field
gradients», Seiten 1232–1234.

Magnetspulenanordnung zur Erzeugung
von linearen magnetischen Gradientenfeldern

Die Erfindung betrifft eine Magnetspulenanordnung zur Erzeugung von linearen magnetischen Gradientenfeldern, vorzugsweise für Magnet-Resonanz-Anordnungen, bestehend aus wenigstens einer Gruppe von vier gleichen, auf einer gedachten Zylindermantelfläche liegenden rechteckförmigen Spulen, von denen jeweils zwei gegenüberliegende Spulenabschnitte parallel zur Zylinderachse verlaufen, wobei die in Umfangsrichtung des Zylinders liegenden Winkelabstände zwischen nebeneinanderliegenden Spulenabschnitten jeweils benachbarter Spulen gleich gross sind, welche in Umfangsrichtung in unterschiedlicher Richtung von dem gleichen Strom durchflossen werden.

Eine derartige Magnetspulenanordnung ist aus dem Aufsatz «Nuclear magnetic resonance zeugmatography for medical imaging» von Ching-Ming Lai, Wylon V. House, Jr. und P.C. Lauterbur, Department of Chemistry, State University of New York at Stony Brook, Stony Brook, N.Y. 11794 8079 Electro/78 Conf.-Record (1978.05) bekannt. Mit ihr lässt sich ein magnetisches Gradientenfeld erzeugen, dessen Feldstärke in einer senkrecht zur Zylinderachse in der Spulenmitte liegenden Ebene entlang einer ersten Achse, die die Zylinderachse schneidet, eine lineare Funktion des Abstandes von der Zylinderachse ist, wobei das magnetische Feld senkrecht auf der ersten Achse steht. Der Magnetfeldgradient weist hierbei eine Konstanz auf, die besser als 1% in einem Zentralbereich der Magnetspulenanordnung ist, dessen radiale Ausdehnung kleiner als ein Viertel des Zylinderdurchtemessers ist. Der Bereich, in dem die Konstanz des Magnetfeldgradienten besser als 1% ist, ist somit gegenüber dem Radius der Magnetspulenanordnung relativ klein. Sind grosse Bereiche von linearen magnetischen Gradientenfeldern erforderlich, beispielsweise für Magnet-Resonanz-Anordnungen zur Untersuchung der nuklearen Spindichteverteilung eines menschlichen Körpers, so sind die Magnetspulenanordnungen entsprechend gross zu dimensionieren, wodurch sich die Kosten für derartige Anordnungen erhöhen.

Aufgabe der Erfindung ist es, eine Magnetspulenanordnung zur Erzeugung von linearen magnetischen Gradientenfeldern zu schaffen, bei der im Verhältnis zum Zylinderdurchmesser die radiale Ausdehnung des Zentralbereiches, in dem die Konstanz des Magnetfeldgradienten besser als 1% ist, erheblich vergrössert ist.

Diese Aufgabe wird gemäss der Erfindung dadurch gelöst, dass entweder die Länge der parallel zur Zylinderachse verlaufenden Spulenabschnitte wenigstens annähernd dem 2,9-fachen des Zylinderradius entspricht und die Winkelabstände wenigstens annähernd 30,3 Grad betragen, oder dass die Länge wenigstens annähernd dem 1,1-fachen des Zylinderradius entspricht und die Winkelabstände wenigstens annähernd 50,9 Grad betragen.

Das durch die vier auf der Zylindermantelfläche angeordneten Spulen im Innern des Zylinders erzeugte Magnetfeld verläuft in der Mitte der Spulen in einer senkrecht zur Zylinderachse liegenden Ebene (Zentralbereich) derart, dass es entlang einer ersten Achse, die die Zylinderachse schneidet, und die symmetrisch zwischen je zwei nebeneinanderliegenden Spulenabschnitten benachbarter Spulen hindurchläuft, linear mit dem Abstand von der Zylinderachse zunimmt, wobei das Magnetfeld senkrecht zu der ersten Achse steht. Der sich ausbildende Magnetfeldgradient wird transversal genannt, da der Vektor, der in die Richtung des grössten Anstieges des Betrages des Magnetfeldes weist, und das Magnetfeld selbst senkrecht zueinander stehen.

Durch die jeweils angegebenen Magnetspulenanordnungen wird nun erreicht, dass der Zentralbereich, in dem eine Konstanz des Magnetfeldgradienten von besser als 1% gefordert wird, im ersten Fall auf etwa 42% und im zweiten Fall auf etwa 32% des Zylinderradius ausgedehnt ist, so dass ein grösserer Objektbereich von einem linearen magnetischen Gradientenfeld bei gleicher Abmessung der Spulenanordnung durchsetzt wird.

Beim Erzeugen von die innere Struktur eines Körpers darstellenden Bildern mit Hilfe von Magnet-Resonanz-Verfahren (spin-imaging) sind einzelne aus unterschiedlichen Bereichen des Körpers kommende Kerninduktionssignale (Kernresonanz- bzw. Bildsignale) durch unterschiedliche Resonanzfrequenzen der Kernspins im Körper voneinander getrennt (siehe z.B. DE-A-27 55 956). Die Resonanzfrequenz wird dabei durch die Stärke eines den Körper durchdringenden Magnetfeldes bestimmt, das sich aus einem homogenen Magnetfeld (Hzo) und weiteren magnetischen Gradientenfeldern mit konstanten Magnetfeldgradienten zusammensetzt. Vorzugsweise verlaufen hierbei zur dreidimensionalen Untersuchung des Körpers zwei senkrecht aufeinander stehende Magnetfeldgradienten jeweils senkrecht zur Richtung des homogenen Magnetfeldes, während ein dritter Magnetfeldgradient in Richtung des homogenen Magnetfeldes verläuft. Könnte man ein einziges Magnetfeld erzeugen, welches an verschiedenen Orten im Körper unterschiedliche Magnetfeldstärken besitzt, so wäre eine einfache Fourieranalyse des Kerninduktionssignals zur Bildrekonstruktion zu verwenden. Da aber die gewünschte Zuordnung zwischen dem Ort und der Resonanzfrequenz praktisch nur ein – oder mit Einschränkungen – zweidimensional möglich ist, können nur ganz bestimmte Bereiche des Körpers nacheinander angeregt werden. Hieraus ergibt sich, dass die Magnetfeldstärke im Körper definiert veränderbar sein muss. Andererseits darf die Variation der Magnetfeldstärke (homogene Magnetfeldstärke plus Feldstärke des Gradientenfeldes) über den Körperbereich nicht zu gross sein, weil damit die Frequenzbandbreite des Kerninduktionssignals zu gross und wegen der Fouriertransformationseigenschaften die verfügbare Dauer des Kerninduktionssignals zu kurz wurde. Man wird also den Betrag der Magnetfeldgradienten möglichst klein wählen, um einen grossen Rauschabstand der Kerninduktionssignale zu erhalten.

Um eindeutige Kerninduktionssignale zu gewinnen, müssen die Magnetfeldgradienten jedoch so gross sein, dass die unvermeidlichen Welligkeiten des magnetischen Feldes nicht zu Doppeldeutigkeiten führen. Die Forderung nach geringer Welligkeit der magnetischen Gradientenfelder bzw. hoher Konstanz der entsprechenden Feldgradienten bedeutet aber, dass alle Multipolkomponenten höherer Ordnung der magnetischen Gradientenfelder sehr klein sein müssen. Somit enthält das gesamte Magnetfeld ausser dem homogenen Magnetfeld ($Hzo$ =const.) nur die linear vom Ort abhängigen, in ihrer Grösse einstellbaren magnetischen Gradientenfelder (mit konstanten Magnetfeldgradienten). Ferner wird durch die Konstanz des magnetischen Feldgradienten das zu erreichende Auflösungsvermögen bezüglich der Kerninduktionssignale bestimmt, das umso grösser ist, je besser die Konstanz des magnetischen Feldgradienten und des homogenen Magnetfeldes ist.

Nach einer vorteilhaften Weiterbildung der Erfindung besitzt die Magnetspulenanordnung für ein Gerät zur Ermittlung einer nuklearen Spindichteverteilung eines Körpers zwei Gruppen von jeweils vier Spulen, von denen je eine Gruppe auf einer gedachten Zylindermantelfläche angeordnet ist, wobei die Zylinderachsen senkrecht aufeinander und senkrecht auf einer Untersuchungsachse stehen, welche jeweils auf halber Länge der Spulen symmetrisch zwischen nebeneinanderliegenden Spulenabschnitten benachbarter Spulen hindurchläuft.

Durch die beiden Gruppen von Spulen wird erreicht, dass in der die beiden Zylinderachsen enthaltenen Ebene zwei senkrecht zueinander in Richtung der Zylinderachsen orientierte Magnetfeldgradienten erzeugbar sind, die im Verhältnis zum Radius gegenüber bekannten Magnetspulenanordnungen über einen erheblich grösseren Bereich konstant sind. Das von den Spulengruppen jeweils erzeugt magnetische Gradientenfeld verläuft dabei parallel zu einer dritten, auf beiden Zylinderachsen senkrecht stehenden Untersuchungsachse.

Unter Zuhilfenahme derartiger Gradientenfelder sind in bekannter Weise Querschnittsbilder von einem nukleare Spins enthaltenden Körper rekonstruierbar, wobei gegenüber bekannten Magnetspulenanordnungen gleicher Abmessung der Bereich, in dem die jeweiligen Magnetfeldgradienten konstant sind, erheblich vergrössert ist, so dass grössere Körperbereiche diagnostiziert werden können.

Nach einer vorteilhaften anderen Weiterbildung der Erfindung weist die Magnetspulenanordnung zusätzlich vier auf einer gedachten Kugeloberfläche angeordnete, ebene Einzelspulen auf, durch deren Mitte und senkrecht zu den Einzelspulen

ebenen die Untersuchungsachse verläuft, wobei die Einzelspulen symmetrisch zum Kugelmittelpunkt, der mit dem Schnittpunkt der Zylinderachsen übereinstimmt, angeordnet sind, und wobei die Einzelspulen, durch die der gleiche Strom fliesst und die gleiche Windungszahlen besitzen, paarweise unter Polarwinkeln zu positiver bzw. negativen Richtung der Untersuchungsachse von wenigstens annähernd 32,0 und 60,4 Grad angeordnet sind.

Die Einzelspulen erzeugen ein weiteres magnetisches Gradientenfeld, welches parallel zur Untersuchungsachse und rotationssymmetrisch zu dieser verläuft. Die Feldstärke hängt dabei linear vom Ort auf der Untersuchungsachse ab. Entsprechendes gilt für Feldlinien, die im Abstand zur Untersuchungsachse verlaufen. Durch die zusätzlichen Einzelspulen ist somit ein magnetischer Feldgradient erzeugbar, der in Richtung des weiteren magnetischen Gradientenfeldes verläuft und der in einem Bereich um den Kugelmittelpunkt herum konstant ist.

Zur Erzeugung eines derartigen Magnetfeldgradienten werden bei bekannten Spulenanordnungen die Einzelspulen von unterschiedlichen Strömen durchflossen, derart, dass das Verhältnis der Ströme des inneren und äusseren Spulenpaares einen bestimmten, sehr genau einzuhaltenden Wert hat, was zusätzlichen schaltungstechnischen Aufwand erfordert und zusätzliche Kosten verursacht.

Mit der vorgeschlagenen Anordnung der vier Einzelspulen, die die gleichen Windungszahlen besitzen, kann erreicht werden, dass bei gleichem Strom in allen Einzelspulen der Magnetfeldgradient in einem Bereich konstant ist, dessen Radius annähernd 43% des Kugelradius entspricht.

Die Zeichnung stellt ein Ausführungsbeispiel der Erfinung dar. Es zeigen

Fig. 1 ein Blockschaltbild eines Gerätes zur Ermittlung der nuklearen Spindichteverteilung in einem Körper,

Fig. 2a eine perspektivisch dargestellte Gruppe von vier Spulen zur Erzeugung eines linearen magnetischen Gradientenfeldes,

Fig. 2b einen Schnitt durch die Gruppe,

Fig. 3 einen Schnitt durch auf einer gedachten Kugeloberfläche angeordnete Einzelspulen,

Fig. 4 eine Magnetspulenanordnung für ein Gerät zur Ermittlung der nuklearen Spindichteverteilung in einem Körper.

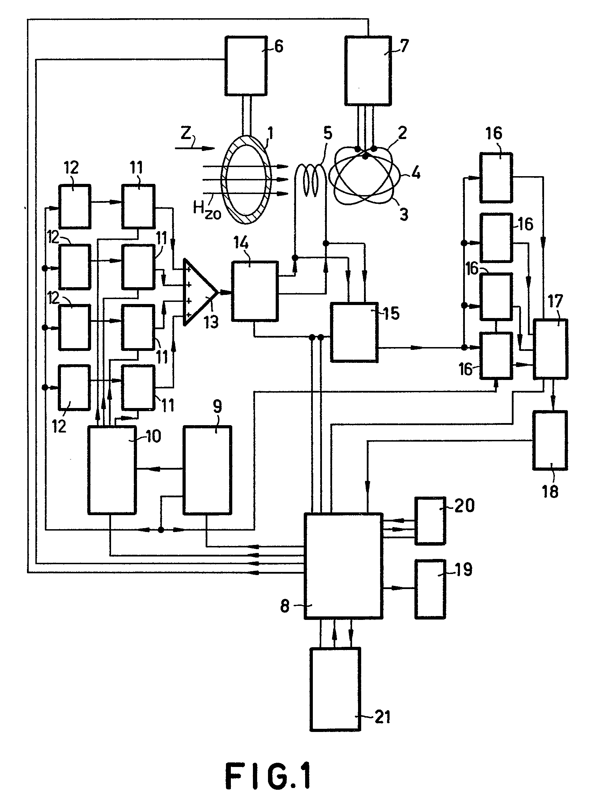

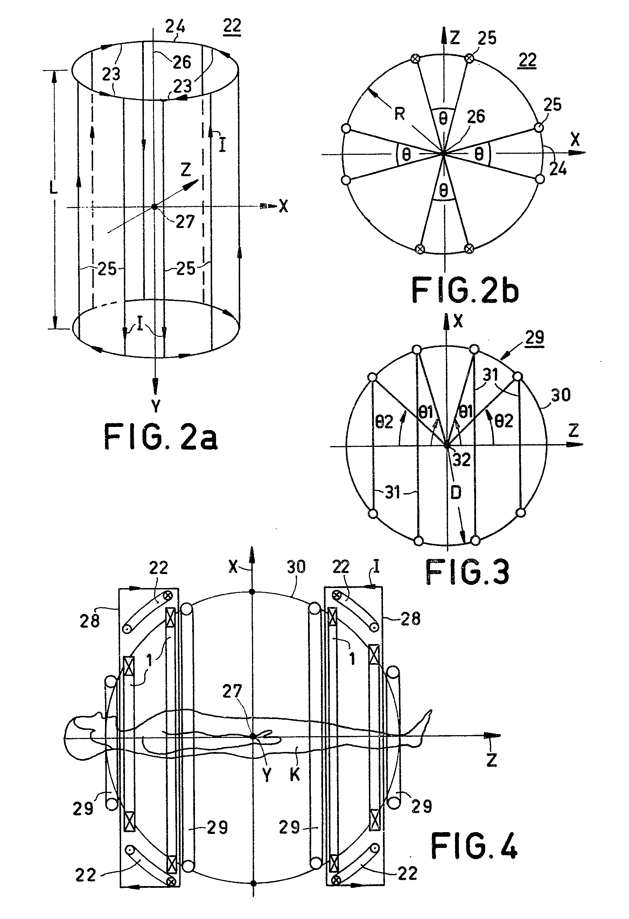

In Fig. 1 ist ein Blockschaltbild eines Gerätes zur Ermittlung der nuklearen Spindichteverteilung eines Körpers dargestellt. Hierbei erzeugt eine Feldspule 1 ein homogenes Magnetfeld $Hzo$, welches in Richtung einer Koordinatenachse Z verläuft und einen Körper K (nicht dargestelllt), der im Innern der Feldspule 1 liegt, durchdringt. Der Körper K, der z.B. auf einem Patiententisch liegt, ist hierbei relativ zur Feldspule 1 in Richtung der Koordinatenachse Z, die die Untersuchungsachse darstellt, verschiebbar. Drei Gradientenspulen 2, 3 und 4 erzeugen hinreichend konstante magnetische Feldgradienten $\partial Hz / \partial x$, $\partial Hz / \partial y$ und $\partial Hz / \partial z$, wobei alle Feldgradienten getrennt einstellbar sind. Die Anregung der Kernspins und Messung der Kerninduktions (Kernresonanz-)-Signale erfolgt durch eine Hochfrequenzspule 5. Sowohl die Gradientenspulen 2, 3 und 4 als auch die Hochfrequenzspule 5 umgreifen den zu untersuchenden Körper K (Fig. 4) im Bereich der Feldspule 1. Die Stromversorgung der Feldspule 1 und der Gradientenspulen 2–4 erfolgt durch Netzgeräte 6 und 7, die unter der Kontrolle einer elektronischen Einheit 8 (Rechen- und Steuergerät) stehen. Die elektronische Einheit 8 steuert den gesamten Messprozess und dient anschliessend zur Rekonstruktion von die innere Struktur des Körpers K darstellenden Bildern.

Das zur Anregung der Kernspins notwendige Hochfrequenzsignal wird von einem Frequenz-Synthetisierer 9 abgeleitet, der ausserdem einen zur Erzeugung eines Modulationssignals erforderlichen Pulsgenerator 10 steuert. Der Pulsgenerator 10 bestimmt über das Modulationssignal mit Hilfe der Modulatoren 11 die Dauer und die Frequenzbandbreite des die Kernspins anregenden Hochfrequenzsignals, welches aus mehreren phasenverschobenen Komponenten bestehen kann, die durch die Phasenschieber 12 erzeugt werden. Im Addierer 13 werden die phasenverschobenen Komponenten des Hochfrequenzsignals addiert. Ein Hochfrequenz- (HF) Leistungsverstärker 14 führt die notwendige HF-Energie zum Anregen der Kernspins der HF-Spule 5 zu, wobei die Impulsleistung je nach Messverfahren und Körpergrösse zwischen 0,05 und 1 Kilo-Watt liegt.

Nach erfolgter Anregung der Kernspins empfängt die HF-Spule 5 das Kerninduktionssignal. Es durchläuft einen weiteren HF-Verstärker 15, anschliessend erfolgt seine Demodulation in den Demodulatoren 16. Ein Analog-Digital-Wandler 17 setzt das demodulierte Kerninduktionssignal in digitale Form um. Ein nachfolgender Signalmittler 18 kann bei Wiederholung des Messzyklus den Rauschabstand unterschiedlicher Kerninduktionssignale verbessern. Ausserdem kann der Signalmittler 18 als digitaler Pufferspeicher benutzt werden.

Mit Hilfe der elektronischen Einheit 8 können dann nach bekannten Verfahren aus den Kerninduktionssignalen die gewünschten Körperbilder erzeugt werden, welche auf einem Monitor 19 darstellbar sind oder auf einen Plattenspeicher 20 in digitaler Form abgelegt werden können. Die Bedienung der gesamten Anlage erfolgt über ein Daten-Ein-Ausgabegerät 21.

In Fig. 2a ist perspektivisch eine erste Gruppe 22 von vier rechteckförmigen Spulen 23 zur Erzeugung eines konstanten Magnetfeldgradienten $\partial Hz/\partial x$ dargestellt, die auf einer gedachten Zylindermantelfläche 24 angeordnet sind. Jede Spule 23 besitzt zwei gerade Spulenabschnitte 25 der Länge L, die parallel zur Zylinderachse 26 verlaufen. Die Zylinderachse 26 stellt hierbei gleichzeitig die Y-Achse eines dreidimensionalen, karthesischen Koordinatensystems XYZ dar, dessen Ursprung 27 auf halber Höhe der Spulen 23 liegt. Die Koordinatenachsen X bzw. Z verlaufen hierbei symmetrisch zwischen nebeneinanderliegenden Spulenabschnitten 25 benachbarter Spulen 23 hindurch, welche in Umfangsrichtung in unterschiedlicher Richtung von dem gleichen Strom I (Pfeil) durchflossen sind. Mittels einer derartigen Gruppe von Spulen 23 ist nun ein magnetischer Feldgradient $\partial Hz/\partial x$ erzeugbar, derart, dass die in Richtung der Koordinatenachse Z weisende magnetische Feldstärke Hz mit hoher Genauigkeit eine lineare Funktion der Ortskoordinate x ist (lineares Gradientenfeld). Hierzu sind die Spulen 23 unter bestimmten Winkelabständen θ auf der Zylindermantelfläche 24 angeordnet.

Die Fig. 2b zeigt hierzu einen Schnitt durch die Gruppe 22, welcher in der X–Z-Ebene liegt. Auf der gedachten Zylindermantelfläche 24, welche einen Abstand R von der Zylinderachse 26 besitzt, liegen alle Spulenabschnitte 25 unter gleichen Winkelabständen θ/2 zu den jeweils nächsten Koordinatenachsen X bzw. Z. Um einen konstanten Magnetfeldgradienten $\partial Hz/\partial x$ von kleiner oder gleich einem Prozent in einem möglichst grossen Bereich in der X-Z-Ebene (Zentralbereich) zu erhalten, beträgt das Verhältnis der Länge L der Spulen 23 zum Radius R annähernd L/R = 2,94. Der Winkelabstand θ/2 der einzelnen Spulenabschnitte 25 zu den jeweils nächsten Koordinatenachsen X bzw. Z beträgt hierbei annähernd 15, 16 Grad (θ = 30,3 Grad). Für diesen Fall ist der Magnetfeldgradient $\partial Hz/\partial x$ bis zu einem Abstand x = 0,42 R von der Zylinderachse 26 in der geforderten Güte konstant.

Beträgt das Verhältnis der Länge L der Spulen 23 zum Radius R annähernd L/R = 1,14 und sind die Winkelabstände θ/2 der einzelnen Spulenabschnitte 25 zu den jeweils nächsten Koordinatenachsen X bzw. Z zu annähernd zu 25,47 Grad (θ = 50,94 Grad) gewählt, so ist der Magnetfeldgradeint $\partial Hz/\partial x$ bis zu einem Abstand x = 0,32 R von der Zylinderachse 26 ebenfalls in der geforderten Güte konstant. Der Radius R kann entsprechend der gewünschten Ausdehnung des konstanten Bereichs des Magnetfeldgradienten $\partial Hz/\partial x$ gewählt werden. Die angegebenen Positionen der Spulenabschnitte 25, die durch den Radius R bzw. den Winkelabstand θ/2 gegeben sind, beziehen sich hierbei auf die Querschnittsmittelpunkte der einzelnen Spulenabschnitte 25. Positionierungsfehler im Winkelabstand von einigen zehntel Grad sind bei den Spulenabschnitten 25 zulässig, ohne dass dadurch die Konstanz des Magnetfeldgradienten die geforderte Güte im angegebenen Bereich unterschreitet. Ebenso wird durch eine Änderung ΔL/L der Länge L der Spulen 23 von wenigen zehntel Prozent die Güte der geforderten Konstanz des Magnetfeldgradienten im angegebenen Bereich nicht verringert.

Zur Erzeugung eines weiteren Magnetfeldgradienten $\partial Hz/\partial y$ ist eine zweite Gruppe 28 (Fig. 4) von vier Spulen 23 vorgesehen, die der Gruppe 22 entspricht. Ihre Stellung relativ zur ersten Gruppe 22 ergibt sich durch Drehung der ersten Gruppe um 90° um die Koordinatenachse Z.

Ferner ist es möglich, die Magnetspulenanordnung, die zwei lineare, transversale Magnetfeldgradienten $\partial Hz/\partial x$ und $\partial Hz/\partial y$ erzeugt, zusätz-

lich mit einer in Fig. 3 dargestellten Magnetspule 29 zur Schaffung eines dreidimensionalen beeinflussbaren Gradientenfeldes zu kombinieren. Das durch die Magnetspule 29 erzeugte magnetische Gradientenfeld verläuft in Richtung der Koordinatenachse Z und besitzt einen linearen, axialsymmetrischen Feldgradienten $\partial Hz/\partial z$. Das bedeutet, dass $\partial Hz/\partial z$ eine annähernd lineare Funktion des Ortes z auf der Koordinatenachse Z ist. Dieser Zusammenhang gilt auch für Punkte, die einen Abstand r von der Koordinatenachse Z haben. Mittels des dreidimensional beeinflussbaren Gradientenfeldes sind dann dreidimensionale Spindichteverteilungen eines Körpers rekonstruierbar.

Die Magnetspule 29 besteht aus vier auf einer gedachten Kugeloberfläche 30 parallel zueinander angeordneten, ebenen Einzelspulen 31, welche senkrecht zur Koordinatenachse Z liegen und symmetrisch zum Kugelmittelpunkt 32, der mit dem Koordinatenursprung 27 zusammenfällt, angeordnet sind. Die Kugeloberfläche 30 besitzt hierbei einen Abstand D vom Kugelmittelpunkt 32.

In bekannten Magnetspulen ist zur Erzeugung eines linearen, axialsymmetrischen Magnetfeldgradienten $\partial Hz/\partial z$ ein ganz bestimmtes, genau einzuhaltendes Verhältnis der Ströme des inneren und äusseren Spulenpaares einzustellen. Oft ist es jedoch wünschenswert, alle Einzelspulen 31 hintereinanderzuschalten und mit gleichen Windungszahlen mit dem gleichen Strom zu betreiben. Hierzu sind die Einzelspulen 31 derart auf der Kugeloberfläche 30 angeordnet, dass die inneren liegenden Einzelspulen 31 unter gleichen Polarwinkeln von $\theta 1 = 60,4$ Grad zur positiven bzw. negativen Richtung der Koordinatenachse Z liegen, während die aussen liegenden Einzelspulen 31 unter gleichen Polarwinkeln von $\theta 2 = 32$ Grad zur positiven bzw. negativen Koordinatenachse Z angeordnet sind. Die auf jeweils einer Seite des Kugelmittelpunktes 32 liegenden Einzelspulen 31 werden dabei in gleicher Richtung vom Strom durchflossen, dessen Richtung aber auf beiden Seiten des Kugelmittelpunktes 32 entgegengesetzt ist.

Auf diese Weise ist ein linearer, axialsymmetrischer Feldgradient $\partial Hz/\partial z$ erzeugbar, dessen Konstanz bis zu einem Abstand r = 0,43 D vom Kugelmittelpunkt 32 besser als 1% ist. Hierbei sind Positionierungsfehler im Polarwinkel von wenigen zehntel Grad zulässig, ohne dass die Konstanz im angegebenen Bereich verschlechtert wird, wobei die Positionierungstoleranz von der gewünschten Konstanz des Feldgradienten abhängig ist.

Die Fig. 4 zeigt eine Anordnung zur Ermittlung der nuklearen Spindichteverteilung eines Körpers K, der im Zentrum eines rechtwinkligen Koordinatensystems XYZ liegt. Durch eine Feldspule 1, die z.B. auf der Kugeloberfläche 30 liegt, wird ein statisches, homogenes Magnetfeld Hzo erzeugt, welches den Körper K parallel zur Koordinatenachse Z durchsetzt. Die Magnetspulenanordnung 22 (erste Gruppe), welche sich parallel zur Koordinatenachse Y erstreckt, erzeugt einen konstanten, transversalen Feldgradienten $\partial Hz/\partial x$, während

die Magnetspulenanordnung 28 (zweite Gruppe), die um 90° gegenüber der Magnetspulenanordnung 22 um die Koordinatenachse Z gedreht ist, einen konstanten, transversalen Feldgradienten $\partial Hz/\partial y$ erzeugt. Beide Magnetspulenanordnungen 22, 28 sind in gleicher Weise und wie in Fig. 2a, b beschrieben, ausgebildet. Zur Erzeugung eines konstanten axialsymmetrischen Feldgradienten $\partial Hz/\partial z$ besitzt die Anordnung nach Fig. 4 eine weitere Magnetspule 29, die ebenfalls auf der gedachten Kugeloberfläche 30 liegt, so dass beispielsweise der Körper K nach dem oben beschriebenen Verfahren untersucht werden kann.

Die zur Bestimmung der nuklearen Spindichteverteilung des Körpers K weiterhin erforderlichen Hochfrequenz-Übertragungs- und Empfangsspulen sind der Übersicht wegen nicht dargestellt.

Die Magnetspulenanordnungen 1, 22, 28 und 29 können selbstverständlich so gross ausgebildet sein, dass ein zu untersuchender Patient in ihrem Innern auch aufrecht sitzen bzw. knien kann. Natürlich ist es weiterhin möglich, mehrere Gruppen 22, 28 und 29 von Spulen derart zu kombinieren, dass entsprechend den Anforderungen verschiedene transversale bzw. axialsymmetrische, senkrecht zueinander stehende Magnetfeldgradienten erzeugbar sind.

**Patentansprüche**

1. Magnetspulenanordnung zur Erzeugung von linearen magnetischen Gradientenfeldern, vorzugsweise für Magnet-Resonanz-Anordnungen, bestehend aus wenigstens einer Gruppe (22; 28) von vier gleichen, auf einer gedachten Zylindermantelfläche (24) liegenden rechteckförmigen Spulen (23), von denen jeweils zwei gegenüberliegende Spulenabschnitte (25) parallel zur Zylinderachse (26) verlaufen, wobei die in Umfangsrichtung des Zylinders liegenden Winkelabstände (8) zwischen nebeneinanderliegenden Spulenabschnitten (25) jeweils benachbarter Spulen (23) gleich gross sind, welche in Umfangsrichtung in unterschiedlicher Richtung von dem gleichen Strom (I) durchflossen werden, dadurch gekennzeichnet, dass entweder die Länge (L) der parallel zur Zylinderachse (26) verlaufenden Spulenabschnitte (25) wenigstens annähernd dem 2,9-fachen des Zylinderradius (R) entspricht und die Winkelabstände ($\theta$) wenigstens annähernd 30,3 Grad betragen, oder dass die Länge wenigstens annähernd dem 1,1-fachen des Zylinderradius entspricht und die Winkelabstände wenigstens annähernd 50,9 Grad betragen.

2. Magnetspulenanordnung nach Anspruch 1, für ein Gerät zur Ermittlung der nuklearen Spindichteverteilung eines Körpers dadurch gekennzeichnet, dass zwei Gruppen (22, 28) von jeweils vier Spulen vorgesehen sind, von denen je eine Gruppe auf je einer gedachten Zylindermantelfläche (24) angeordnet ist, wobei die Zylinderachsen (X, Y) senkrecht aufeinander und senkrecht auf einer Untersuchungsachse (Z) stehen, welche jeweils auf halber Länge der Spulen symmetrisch zwischen nebeneinander liegenden Spulenabschnitten (25) benachbarter Spulen hindurchläuft.

3. Magnetspulenanordnung nach Anspruch 2, dadurch gekennzeichnet, dass sie zusätzlich vier auf einer gedachten Kugeloberfläche (30) angeordnete ebene Einzelspulen (31) besitzt, durch deren Mitte und senkrecht zu den Einzelspulenebenen die Untersuchungsachse (Z) verläuft, wobei die Einzelspulen symmetrisch zum Kugelmittelpunkt (32), der mit dem Schnittpunkt der Zylinderachsen XY übereinstimmt, angeordnet sind, und dass die Einzelspulen 31, durch die der gleiche Strom fliesst und die gleiche Windungszahlen besitzen, paarweise unter Polarwinkeln ($\theta$1, $\theta$2) zur positiven bzw. negativen Richtung der Untersuchungsachse von wenigstens annähernd 32,0 und 60,4 Grad angeordnet sind.

**Revendications**

1. Montage de bobines magnétiques pour l'obtention de champs à gradient magnétique linéaire, de préférence pour des dispositifs à résonance magnétique comportant au moins un groupe (22, 28) de quatre bobines (23) semblables de forme rectangulaire disposées sur une enveloppe cylindrique imaginaire (24) parmi lesquelles deux sections de bobines (25) opposées sont chaque fois parallèles à l'axe (26) du cylindre, les distances angulaires ($\theta$) dans le sens périphérique du cylindre entre des sections (25) contiguës de bobines (23) voisines étant chaque fois les mêmes, les bobines étant parcourues dans le sens périphérique par le même courant (I) dans des directions différentes, caractérisé en ce que ou bien la longueur (L) des sections de bobines (25) s'étendant parallèlement à l'axe (26) du cylindre correspond au moins à peu près à 2,9 fois le rayon (R) du cylindre et les distances angulaires ($\theta$) sont au moins d'environ 30,3 °, ou bien la longueur correspond au moins à peu près à 1,1 fois le rayon du cylindre et les distances angulaires sont d'au moins à peu près 50,9 °.

2. Montage de bobines magnétiques suivant la revendication 1 pour un appareil servant à déterminer le spectre de densité de spin nucléaire d'un corps, caractérisé en ce que deux groupes (22, 28) de quatre bobines chacun sont prévus, un groupe étant chaque fois disposé sur une enveloppe cylindrique imaginaire (24), les axes (X, Y) du cylindre s'étendant perpendiculairement l'un à l'autre et perpendiculairement à un axe d'examen (Z) qui passe chaque fois à mi-longueur des bobines symétriquement entre des sections (25) contiguës de bobines voisines.

3. Montage de bobines magnétiques suivant la revendication 2, caractérisé en ce qu'il comporte en outre quatre bobines individuelles planes (31) disposées sur une surface sphérique imaginaire (30), l'axe d'examen (Z) passant par leur milieu et s'étendant perpendiculairement à leurs plans, étant entendu que les bobines individuelles sont disposées symétriquement par rapport au centre de la sphère (32) qui correspond au point d'intersection des axes (X, Y) de cylindre et que les bobines individuelles (31) qui sont parcourues par le même courant et présentent un même nombre de spires sont disposées par paires, en formant avec le sens positif ou négatif de l'axe d'examen des angles polaires ($\theta$1, $\theta$2) d'au moins à peu près 32,0 et 60,4 °.

**Claims**

1. A magnet coil arrangement for generating linear magnetic gradient fields, preferably for magnetic resonance arrangements, consisting of at least one group (22; 28) of four identical rectangular coils (23) which are situated on an imaginary cylinder surface (24), each time two oppositely situated coil sections (25) thereof extending parallel to the cylinder axis (26), the angular distances (8) between adjacent coil sections (25) of each time neighbouring coils (23) being equal in the circumferential direction of the cylinder, the same current (I) flowing through said coils in different directions in the circumferential direction, characterized in that either the length (L) of the coil sections (25) extending paralllel to the cylinder axis (26) corresponds to at least approximately 2.9 times the cylinder radius (R) and that the angular distances ($\theta$) amount to at least approximately 30.3 degrees, or that the length corresponds to at least approximately 1.1 times the cylinder radius and the angular distances amount to at least approximately 50.9 degrees.

2. A magnet coil arrangement as claimed in Claim 1 for an apparatus for determining the nuclear spin density distribution of a body, characterized in that there are provided two groups (22, 28) of four coils each, each group being accommodated each time on an imaginary cylinder surface (24), the cylinder axes (X, Y) extending perpendicularly to one another and perpendicularly to an examination axis (Z) which extends eacht time symmetrically between adjacent coil sections (25) of neighbouring coils at half the length of the coils.

3. A magnet coil arrangement as claimed in Claim 2, characterized in that it additionally comprises four flat single coils (31) which are arranged on an imaginary spherical surface (30), the examination axis (Z) extending through the centre thereof and perpendicularly to the planes of the single coils, the single coils being symmetrically arranged with respect to the sphere centre (32) which corresponds to the point of intersection of the cylinder axes (X, Y), the single coils (31) wherethrough the same current flows and which comprise the same number of turns being pair-wise arranged at pole angles ($\theta$1, $\theta$2) of at least approximately 32.0 and 60.4 degrees with respect to the positive direction and the negative direction, respectively of the examination axis.

1/2

FIG.1

FIG. 2a

FIG. 2b

FIG. 3

FIG. 4